# FASCICULE DE BREVET EUROPEEN

(11) **EP 0 679 388 B1**
(45) Date de publication et mention de la délivrance du brevet: **07.10.1998**
(21) Numéro de dépôt: 95400651.6
(22) Date de dépôt: 23.03.1995
(51) Int. Cl.: A61K 7/48

(54) **Composition cosmetique et/ou dermatologique notamment pour dépigmenter la peau, ses utilisations**
Kosmetische und/oder dermatologische Zusammensetzung, insbesondere zur Depigmentierung der Haut, und ihre Verwendungen
Cosmetic and/or dermatologic skin depigmentation composition and its use

(30) Priorité: 18.04.1994 FR 9404603
(43) Date de publication de la demande: 02.11.1995
(73) Titulaire: L'OREAL, 75008 Paris (FR)
(72) Inventeur: Arnaud-Sebillotte, Laurence, F-94000 Creteil (DE); Segot, Evelyne, F-94130 Nogent-sur-Marne (FR)
(74) Mandataire: Lhoste, Catherine

(56) Documents cités:
- WO-A-93/07856
- FR-A- 2 681 245
- FR-A- 2 698 004

## Description

L'invention se rapporte à une composition cosmétique et/ou dermatologique destinée plus spécialement à la dépigmentation de la peau, aussi bien du visage que du corps humain voire même du cuir chevelu.

L'invention se rapporte aussi à une utilisation de cette composition pour le traitement cosmétique de la peau et en particulier pour dépigmenter la peau, à l'utilisation de cette composition pour la préparation d'une pommade ou d'un onguent destiné au traitement dermatologique de la peau et à un procédé de traitement cosmétique de la peau.

A différentes périodes de leur vie, certaines personnes voient apparaître sur la peau et plus spécialement sur les mains des taches plus foncées et/ou plus colorées, conférant à la peau une hétérogénéité. En général, ces taches sont dues à une production importante de mélanine dans l'épiderme et/ou le derme de la peau.

Ces taches peuvent être liées à plusieurs phénomènes et plus spécialement au vieillissement. Dans certains cas, ces taches peuvent devenir cancéreuses. Aussi, on cherche de plus en plus à diminuer, voire éliminer, ces taches.

Les compositions classiquement utilisées dans les domaines cosmétique et/ou dermatologique sont des émulsions eau-dans-huile (E/H), des émulsions huile-dans-eau (H/E), ou des gels aqueux, dans lesquels il est souvent difficile, voire même impossible d'incorporer des actifs dépigmentants ou antipigmentants comme l'acide kojique, l'acide caféique, l'acide salicylique et ses dérivés mais aussi d'autres actifs dépigmentants ou antipigmentants, utilisés dans différents types de traitement comme la lutte contre le vieillissement, ou dans certaines maladies de la peau (mélasmes, lentigines).

En général, ces actifs ont tendance à recristalliser ou à se dégrader. Il s'ensuit une perte d'efficacité plus ou moins importante de ces compositions, selon le degré de recristallisation et/ou de dégradation, ce qui va à l'encontre de l'objectif recherché. En outre, cette recristallisation ou dégradation peut modifier la stabilité globale de ces compositions ainsi que leur aspect, ce qui peut détourner l'utilisateur de ces compositions à traitement spécifique. Par ailleurs leur dissolution dans une composition quelconque nécessite souvent de chauffer cette dernière, ce qui est relativement gênant pour des actifs sensibles à la chaleur comme les vitamines, certains extraits de plante ou certaines protéines.

Pour solubiliser certains de ces actifs, il est connu d'utiliser des émulsions E/H ou H/E dans lesquelles la phase aqueuse présente un pH acide. Pour que ces émulsions soient stables (non séparation des phases aqueuse et huileuse), il est nécessaire d'utiliser des émulsionnants (ou tensioactifs). Malheureusement, ces tensioactifs sont souvent irritants pour la peau. En outre ces émulsions manquent souvent de fraîcheur à l'application, ce qui peut gêner leurs utilisations pendant les périodes chaudes de l'année et/ou dans les pays chauds. Un gel aqueux est beaucoup plus apprécié dans ces conditions d'utilisation. Mais sa trop grande quantité d'eau ne permet pas d'y introduire ces actifs.

Le document WO-A-93/07856 décrit un gel aqueux de pH inférieur à 4 contenant un polyacrylamide non ionique. Le problème de la solubilisation d'actifs, notamment d'actifs dépigmentants, n'est pas abordé dans ce document, et les compositions décrites comportent toutes un minimum de plus de 50 % d'eau. De telles proportions d'eau sont trop importantes pour permettre une bonne solubilisation des actifs dans les solvants.

Il subsiste donc le besoin d'une composition stable ayant l'aspect d'un gel, utilisable notamment dans les domaines cosmétique et/ou dermatologique permettant une solubilisation suffisante des actifs généralement utilisés dans ces domaines en vue d'une efficacité maximum.

De façon surprenante, la demanderesse a trouvé qu'il était possible de solubiliser une grande quantité d'actif dans une composition cosmétique et/ou dermatologique ayant toutes les propriétés d'un gel.

Ainsi, l'invention a pour objet une composition contenant un milieu hydrophile acide et au moins un agent gélifiant formé d'un copolymère anionique réticulé d'acrylamide, d'acide 2-acrylamino 2-méthyl propane sulfonique et d'au moins un composé à polyinsaturation oléfinique servant d'agent de réticulation, caractérisée en ce que le milieu hydrophile est un milieu, riche en solvant organique et présente un pH au plus égal à 4, ce milieu renfermant une quantité d'eau représentant au plus 45% du poids total de la composition, le gélifiant conférant à la composition un aspect macroscopiquement homogène.

La composition de l'invention a une certaine consistance et/ou tenue ; elle n'est pas filante, c'est-à-dire qu'elle ne forme pas de fil lorsqu'on la prend au doigt. Elle se présente plus spécialement sous forme d'un gel.

Cette composition, contrairement aux émulsions, ne contient peu ou pas de tensioactifs. Par ailleurs, contrairement aux processus d'obtention classiques des compositions gélifiées ou émulsionnées, il n'est pas nécessaire de chauffer pour former la composition. Ceci facilite donc sa fabrication et permet d'utiliser des actifs sensibles à la chaleur comme les vitamines ou les extraits de végétaux. Il s'ensuit donc une efficacité améliorée par rapport aux compositions obtenues par chauffage.

Cette composition est particulièrement bien adaptée à la solubilisation d'actifs utilisés dans les domaines cosmétique et dermatologique. Aussi, la composition de l'invention contient, en outre, au moins un actif choisi notamment parmi les actifs dépigmentants et kératolytiques.

Comme actifs solubilisables dans la composition de l'invention, on peut citer l'acide ascorbique, l'acide kojique, l'acide citrique, l'acide caféique, l'acide salicylique et ses dérivés (par exemple acide n-octanoyl-5 ou décanoyl-5-salicylique), les α-hydroxyacides tels que l'acide lactique, l'acide méthyllactique, l'acide glucuronique, l'acide glycolique, l'acide pyruvique, l'acide 2-hydroxybutanoïque, l'acide 2-hydroxypentanoïque, l'acide 2-hydroxyhexanoïque, l'acide 2-hydroxyheptanoïque, l'acide 2-hydroxyoctanoïque, l'acide 2-hydroxynonanoïque, l'acide 2-hydroxydécanoïque, l'acide 2-hydroxyundécanoïque, l'acide 2-hydroxydodécanoïque, l'acide 2-hydroxytétradécanoïque, l'acide 2-hydroxyhexadécanoïque, l'acide 2-hydroxyoctadécanoïque, l'acide 2-hydroxytétraécosanoïque, l'acide 2-hydroxyeïcosanoïque, l'acide mandélique, l'acide benzoïque, l'acide phényllactique, l'acide gluconique, l'acide galacturonique, l'acide citrique, l'acide aleuritique, l'acide ribonique, l'acide tartronique, l'acide tartrique, l'acide malique, l'acide fumarique, l'acide rétinoïque et ses dérivés tels que le rétinol et les esters de rétinol, l'acide benzène 1,4-di(3-méthylidène 10-camphosulfonique). On peut aussi, utiliser tous les composés naturels ou synthétiques contenant de tels acides, comme les extraits végétaux et plus spécialement les extraits de fruits. On peut aussi solubiliser les dérivés xanthiques (caféine, théophylline), l'acide β-glycyrrhétinique, l'acide asiatique, l'octopirox, ou encore l'acide rétinoïque.

Il est tout à fait surprenant d'obtenir une composition parfaitement stable, avec le copolymère de l'invention, connu pour épaissir un milieu hydrophile contenant une grande quantité d'eau. En effet, rien ne permettait de prévoir que cet épaississant puisse gélifier et stabiliser efficacement une composition aussi pauvre en eau, et en particulier contenant au plus 30 % d'eau. Par ailleurs, ce copolymère favorise la solubilisation des actifs ci-dessus.

Certes, il est connu de stabiliser des compositions cosmétiques avec des gélifiants comme les polymères naturels du type dérivés de cellulose (hydroxypropyl cellulose, hydroxypropyl méthylcellulose, hydroxyéthylcellulose alkylé), les dérivés de polyolosides (hydroxypropylguar), ou comme les polymères de synthèse tels que le copolymère métho-chlorure de vinylimidazolinium/vinyl pyrrolidone et le copolymère ammonium acrylate/acrylonitrogène. Mais ces gélifiants, qui ont été testés par la demanderesse, ne sont nullement appropriés aux compositions acides et riches de l'invention.

Par composition stable, il faut comprendre une composition restant homogène et dans laquelle le ou les actifs ne recristallisent et/ou ne se dégradent pas, notamment pendant au moins 2 mois à 45 ° C.

De façon avantageuse, le pH de la phase aqueuse est choisi inférieur ou égal à 3, et en pratique choisi dans la gamme allant de 1 à 3.

Le ou les gélifiants de l'invention sont notamment des copolymères anioniques réticulés, substantiellement solubles dans l'eau et constitués de motifs résultant de la réaction entre (i) l'acrylamide, (ii) l'acide 2-acrylamino 2-méthyl propane sulfonique (ci-après dénommé par commodité AMPS) et (iii) au moins un composé à polyinsaturation oléfinique (servant d'agent de réticulation), ce composé à polyinsaturation oléfinique étant présent dans le copolymère à une concentration allant de 0,06 à 1 millimole par mole de l'ensemble des motifs monomères.

Les copolymères ci-dessus peuvent ainsi être obtenus classiquement selon la technique dite de polymérisation en émulsion à partir des trois différents comonomères rentrant dans leur constitution.

Les monomères à polyinsaturation oléfinique utilisés comme agent de réticulation pour la préparation des copolymères conformes à l'invention sont de préférence choisis dans le groupe constitué par le méthylène bis-acrylamide, l'allyle-sucrose et le pentaérythritol. Encore plus préférentiellement, on fait appel au méthylène bis-acrylamide.

Le rapport, exprimé en moles %, entre l'acrylamide et l'AMPS est généralement choisi de 85/15 à 15/85, de préférence de 70/30 à 30/70, encore plus préférentiellement de 65/35 à 35/65, et plus particulièrement encore de 60/40 à 40/60. En outre, l'AMPS est généralement au moins partiellement neutralisé sous la forme d'un sel, par exemple par de la soude, de la potasse, ou une amine à faible poids moléculaire telle que la triéthanolamine, ou leurs mélanges.

Un copolymère réticulé particulièrement préféré pour la mise en oeuvre de l'invention correspond à celui tel que préparé selon l'exemple 1 de la demande de brevet EP-A- 0 503 853 précitée, et qui se présente alors sous la forme d'une émulsion inverse eau-dans-huile. Plus précisément, ce copolymère est constitué de 60% en moles d'acrylamide et de 40% en moles de sel de sodium d'AMPS, et il est réticulé par du méthylène bis-acrylamide à raison de 0,22 millimole par mole du mélange total de monomères. L'émulsion inverse finale eau-dans-huile contient, quant à elle, et de préférence, environ 40% en poids de copolymère réticulé tel que défini ci-avant et de l'ordre de 4% en poids d'un alcool gras éthoxylé ayant une valeur balance lipophile/hydrophile, appelée en général HLB, d'environ 12,5.

Il est possible d'associer à ce polymère d'autres gélifiants hydrosolubles tels que les gommes de cellulose, les polyuréthannes, les dérivés acryliques comme par exemple le polyméthacrylate de glycéryle à 2 % dispersé dans un mélange eau/glycérine vendu sous le nom NORGEL ou LUBRAGEL par la société GUARDIAN.

La quantité de copolymère anionique, selon l'invention, est choisie, en matière active, par exemple de 0,01 % à 5 % du poids total de la composition et de préférence de 0,1 % à 3 % et mieux encore de 1 % à 2 % ; ceci correspond à environ 0,1 % à 10 % du poids total de la composition et de préférence de 1 % à 8 % et mieux de 3 % à 5 % pour la formulation commerciale du copolymère.

La quantité, en matière active, de gélifiants additionnels est choisie par exemple de 0 % à 10 % du poids total de la composition, de préférence de 0,1 % à 6 % et mieux de 0,5 % à 3 %.

Les solvants utilisables dans l'invention sont des solvants hydrophiles associés éventuellement à des solvants lipophiles ou à la fois hydrophiles et lipophiles. En particulier, ces solvants sont des solvants cosmétiquement et/ou dermatologiquement acceptables (tolérance, toxicologie et toucher acceptables).

Les solvants hydrophiles peuvent représenter de 5 % à 90 % du poids total de la composition et de préférence de 20 % à 70 % et mieux de 50 % à 70 %. Ces solvants hydrophiles sont par exemple des mono-alcools inférieurs linéaires ou ramifiés ayant de 1 à 8 atomes de carbone comme l'éthanol, le propanol, le butanol, l'isopropanol, l'isobutanol ; des polyéthylène glycols ayant de 6 oxydes d'éthylène à 80 oxydes d'éthyléne ; des polyols tels que le propylène glycol, l'isoprène glycol, le butylène glycol, le glycérol, le sorbitol ; les mono- ou di-alkyle d'isosorbide dont les groupements alkyle ont de 1 à 5 atomes de carbone comme le diméthyl isosorbide ; les éthers de glycol comme le diéthylène glycol mono-méthyl ou mono-éthyl éther et les éthers de propylène glycol comme le dipropylène glycol méthyl éther.

La composition contient de préférence au moins 10 % en poids d'alcools inférieurs ayant de 2 à 8 atomes de carbone.

Comme solvants à la fois lipophiles et hydrophiles, on peut citer des polyols tels que l'isoprène glycol ; des dérivés de polypropylène glycol (PPG) tels que les esters de polypropylène glycol et d'acide gras, de PPG et d'alcool gras comme le PPG-23 oléyl éther et le PPG-36 oléate. Ces solvants sont utilisés en combinaison avec les solvants hydrophiles avec une concentration allant de 0 % à 50 % du poids total de la composition et de préférence de 0 % à 30 % et mieux de 0 à 10 %.

On peut éventuellement utiliser des solvants lipophiles associés aux solvants hydrophiles et/ou à la fois hydrophiles et lipophiles. Ces solvants lipophiles représentent de 0 % à 50 % du poids total de la composition et de préférence de 0 % à 30 % et mieux de 0 à 10 %. Ces solvants sont en particulier des esters gras tels que l'adipate de diisopropyle, l'adipate de dioctyle, le malate de dioctyle, l'isostéarate de glycéryle, l'acétostéarate d'octyle, l'octanoate de cétyle, le sébacate de diisopropyle ; des alcools de Guerbet (ou 2-alkyl-1-alcanols) de structure générale R₁-CHR₂-CH₂OH avec R₁ et R₂ identiques ou différents représentant un atome d'hydrogène ou un radical alkyle ayant de 1 à 10 atomes de carbone comme l'octyldodécanol, l'hexyldécanol, le cyclododécanol.

Afin que la composition de l'invention soit plus agréable à utiliser (plus douce à l'application, plus nourrissante, plus émolliente), il est possible d'ajouter une ou plusieurs huiles de préférence solubles dans le milieu hydrophile, c'est-à-dire dans l'eau ou le solvant. De préférence, la composition est exempte d'huile insoluble dans le milieu hydrophile afin d'obtenir une structure gel.

Les huiles solubles dans l'eau ou dans le milieu hydroalcoolique représentent de 0 % à 50 % du poids total de la composition, de préférence de 0 % à 30% et mieux de 1 % à 15 %.

Les huiles partiellement solubles dans le solvant, utilisables dans l'invention, sont par exemple les cyclométhicones (cyclohexa-, cyclopenta- ou cyclotétra-diméthylsiloxane), les polydiméthyl siloxanes volatils, linéaires (à 2, 3, ou 4 fonctions siloxane) ou encore les phényldiméthyl siloxanes.

Les huiles solubles dans l'eau, utilisables dans l'invention, sont par exemple les silicones hydrosolubles telles que les :
- polydiméthyl siloxanes oxyéthylénés à motifs amide comme par exemple ceux vendus sous le nom Silwax AX ou Silwax DCA-100 par la société Siltech ;
- polydiméthyl siloxanes oxyéthylénés à groupements stéarate comme par exemple ceux vendus sous le nom Silwax WD-IS par la société Siltech ;
- polydiméthyl siloxanes oxyéthylénés comme par exemple ceux vendus sous le nom Belsil DMC 6038 par la société Wacker ;
- stéaroxy polydiméthyl siloxanes comme par exemple ceux vendus sous le nom Belsil SDM 6022 par la société Wacker.

On peut aussi utiliser des huiles insolubles dans le milieu hydroalcoolique. Ces huiles représentent de 0 % à 20 % et de préférence de 1 % à 15 %.

Ces huiles insolubles sont par exemples :
- les huiles minérales telles que l'huile de paraffine et de vaseline,
- les huiles d'origine animale telles que le perhydrosqualène,
- les huiles d'origine végétale telles que l'huile d'amande douce, l'huile d'avocat, l'huile de ricin, l'huile d'olive, l'huile de jojoba, l'huile de sésame, l'huile d'arachide, l'huile de pépins de raisin, l'huile de colza, l'huile de coprah, l'huile de noisette, le beurre de karité et sa fraction liquide, l'huile de palme, l'huile de noyau d'abricot, l'huile de calophyllum, l'huile de son de riz, l'huile de germe de maïs, l'huile de germe de blé, l'huile de soja, l'huile de tournesol, l'huile d'onagre, l'huile de carthame, l'huile de passiflore et l'huile de seigle,
- les huiles synthétiques telles que l'huile de purcellin, les esters gras dont le myristate de butyle, le myristate d'isopropyle, le stéarate d'héxadécyle, le stéarate d'isopropyle, le stéarate d'octyle, le stéarate d'isocétyle, l'oléate de décyle, le laurate d'héxyle, le dicaprylate de propylène glycol et les esters dérivés d'acide lanolique tels que le lanolate d'isopropyle, le lanolate d'isocétyle et les isoparaffines, les huiles fluorées et perfluorées, et enfin les huiles siliconées, volatiles ou non.

Les huiles utilisées peuvent également contenir un ou plusieurs actifs cosmétiques et/ou thérapeutiques lipophiles, notamment ceux utilisés de manière habituelle dans la fabrication et l'obtention des compositions cosmétiques et/ou pharmaceutiques. Ces actifs peuvent être, par exemple, des agents anti-radicaux libres ; des céramides ; des filtres solaires (notamment ultraviolets) tels que le paraméthoxycinnamate de 2-éthylhexyle et en particulier celui commercialisé sous la dénomination "Parsol MCX" par la Société GIVAUDAN ou la 2-hydroxy-4-méthoxybenzophénone et en particulier celle vendue sous la dénomination commerciale "Uvinul M40" par la Société BASF ; des répulsifs pour insectes tels que le cétyl-3 éthylaminopropionate de n-butyle ou le diéthylamide-N,N-caprylique ; des agents amincissants tels que le nicotinate de D-L α-tocophérol, l'extrait huileux de ginseng (Panax Ginseng), l'extrait huileux de lierre grimpant (Hedera Helix), l'extrait huileux de fleurs sèches d'arnica (Arnica montana L) et l'extrait huileux d'algues (Fucus Vesiculosus). Il va de soi que la liste ci-dessus d'actifs lipophiles susceptibles d'être introduits dans la composition de l'invention n'est nullement exhaustive.

Les huiles peuvent également contenir, selon l'application envisagée, un ou plusieurs additifs de formulation lipophiles tels que des agents conservateurs, antioxydants ou émollients, des huiles parfumées voire même des parfums. L'invention a encore pour objet l'utilisation de la composition ci-dessus pour traiter la peau du visage et/ou du corps humain et notamment pour dépigmenter la peau du visage et/ou du corps humain et plus spécialement les mains.

L'invention se rapporte aussi à un procédé de traitement cosmétique, par voie topique, de la peau, qui consiste à appliquer sur la peau, du visage et/ou du corps humain une composition telle que définie précédemment. Le type de traitement est fonction du ou des actifs solubilisés dans la composition.

Plus spécialement, l'invention se rapporte à un procédé pour dépigmenter la peau du visage et/ou du corps humain, consistant à appliquer sur la peau une composition telle définie ci-dessus contenant au moins un agent dépigmentant solubilisé.

L'invention a encore pour objet une utilisation de la composition ci-dessus pour préparer une pommade ou un onguent destiné à traiter thérapeutiquement le visage et/ou le corps humain, y compris les mains.

L'invention a encore pour objet une utilisation d'un copolymère tel que défini précédemment, pour solubiliser un actif dans une composition homogène macroscopiquement, contenant un milieu hydrophile acide, riche en solvant organique et présentant un pH au plus égal à 4, ce milieu renfermant une quantité d'eau représentant au plus 45% du poids total de la composition et/ou pour gélifier ladite composition.

La description qui suit est donnée à titre illustratif et non limitatif. Les concentrations des différents constituants entrant dans les compositions exemplifiées ci-après, sont données en pourcentage pondéral. Ces différentes compositions ont été obtenues selon le mode opératoire suivant.

On solubilise le ou les actifs dans le solvant ou le mélange de solvants choisi (phase A), puis on gélifie avec le copolymère (phase C) par simple homogénéisation à température ambiante au moyen d'une turbine à hélice. Les huiles émollientes (phase B) sont ensuite incorporées, toujours à température ambiante de la même façon que pour le copolymère. Cette mise en oeuvre est vraiment simple à réaliser, ce qui est, pour une fabrication en série, un gros avantage, diminuant le prix de revient des produits finaux.

Le copolymère correspond, dans tous exemples ci-après au produit préparé selon l'exemple 1 du document EP-A-503853 cité précédemment ; ce produit se présente sous la forme d'une émulsion de type eau-dans-huile et contenant 40 % environ d'un copolymère réticulé acrylamide (60 % en mole)/AMPS sodé (40 % en mole)/méthylène bis-acrylamide (0,22 mmol/mol), et 4 % en poids environ d'un alcool gras éthoxylé ayant une valeur de HLB d'environ 12,5.

Les compositions obtenues sont soit fluides et non filantes, soit avec un seuil d'écoulement tel que le produit ne s'écoule pas sous son propre poids. Elles sont onctueuses et peuvent avoir un aspect de gel transparent ou translucide ou l'aspect d'une crème opaque. Ces compositions sont toutes des compositions dépigmentantes, mais il va de soi que l'invention est d'application beaucoup plus générale comme indiqué précédemment.

### EXEMPLE 1 : GEL DEPIGMENTANT

| ***Phase A*** | |
|---|---|
| Eau déminéralisée | qsp 100 % |
| Alcool éthylique à 96 ° dans de l'eau | 30 % |
| Polyéthylène glycol 8 fois oxyéthyléné | 30 % |
| Acide kojique | 1 % |
| Acide caféique | 1,2 % |
| Acide n-octanoyl-5-salicylique | 1,5 % |

| ***Phase B*** | |
|---|---|
| Polydiméthyl siloxane oxyéthyléné (Belsil DMC 6038) | 2 % |

| ***Phase C*** | |
|---|---|
| Produit de l'exemple 1 du EP-A-503853 | 5 % |

Le produit obtenu a l'aspect d'un gel translucide, lisse, brillant et stable. Son pH est de 3,1. Il peut être appliqué chaque jour sur les taches du visage et du cou en vue de les diminuer.

### EXEMPLE 2 : GEL DEPIGMENTANT

| ***Phase A*** | |
|---|---|
| Eau déminéralisée | qsp 100 % |
| Alcool éthylique à 96 ° dans de l'eau | 30 % |
| Polyéthylène glycol 8 fois oxyéthyléné | 30 % |
| Acide kojique | 1 % |
| Acide caféique | 0,2 % |
| Acide salicylique | 1 % |

| ***Phase B*** | |
|---|---|
| Cyclohexadiméthylsiloxane | 5 % |

| ***Phase C*** | |
|---|---|
| Produit de l'exemple 1 du EP-A-503853 | 4 % |

| ***Phase D*** | |
|---|---|
| Polyméthacrylate de glycéryle à 2 % dans un mélange eau/glycérine (Norgel) | 20 % |

La phase D est introduite dans le mélange A+B+C à température ambiante par simple homogénéisation.

Le produit obtenu a l'aspect d'un gel translucide, lisse, brillant et stable. Son pH est de 3,1. Il peut être appliqué une ou deux fois par jour sur les taches du visage et/ou du cou en vue de les diminuer.

### EXEMPLE 3 : GEL ANTI-TACHES POUR LES MAINS

| ***Phase A*** | |
|---|---|
| Eau déminéralisée | qsp 100 % |
| Octyldodécanol | 30 % |
| Polyéthylène glycol 8 fois oxyéthyléné | 30 % |
| Acide kojique | 1 % |
| Acide caféique | 1,2 % |
| Acide n-octanoyl-5-salicylique | 0,5 % |

| ***Phase B*** | |
|---|---|
| Huile de macadamia | 2 % |

| ***Phase C*** | |
|---|---|
| Produit de l'exemple 1 du EP-A-503853 | 2,5 % |

Le produit obtenu a l'aspect d'une crème opaque, lisse, brillante et stable. Son pH est de 3,1. Il peut être appliqué chaque jour sur les taches des mains.

### EXEMPLE 4 : GEL ANTI-TACHES POUR LES MAINS

| ***Phase A*** | |
|---|---|
| Eau déminéralisée | qsp 100 % |
| Alcool éthylique à 96 °dans l'eau | 30 % |
| Polyéthylène glycol 8 fois oxyéthyléné | 30 % |
| Acide caféique | 1 % |

| ***Phase B*** | |
|---|---|
| Huile d'amande douce | 2 % |

| ***Phase C*** | |
|---|---|
| Produit de l'exemple 1 du EP-A-503853 | 2,5 % |

Le produit obtenu a l'aspect d'un gel/crème opaque, lisse, brillant et stable. Son pH est de 3,1. Il peut être appliqué chaque jour sur les taches des mains

### EXEMPLE 5 : GEL ANTI-TACHES POUR LES MAINS

| ***Phase A*** | |
|---|---|
| Eau déminéralisée | qsp 100 % |
| Ethanol | 30 % |
| Propylène glycol | 30 % |
| Acide kojique | 2 % |
| Acide salicylique | 1 % |

| ***Phase C*** | |
|---|---|
| Produit de l'exemple 1 du EP-A-503853 | 2,5 % |

Le produit obtenu a l'aspect d'un gel translucide, lisse, brillant et stable. Il peut être appliqué chaque jour sur les taches des mains.

## Revendications

1. Composition contenant un milieu hydrophile acide et au moins un agent gélifiant formé d'un copolymère anionique réticulé d'acrylamide, d'acide 2-acrylamino 2-méthyl propane sulfonique et d'au moins un composé à polyinsaturation oléfinique servant d'agent de réticulation, caractérisée en ce que le milieu hydrophile est un milieu, riche en solvant organique et présente un pH au plus égal à 4, ce milieu renfermant une quantité d'eau représentant au plus 45% du poids total de la composition, le gélifiant conférant à la composition un aspect macroscopiquement homogène.

2. Composition selon la revendication 1, caractérisée en ce que la composition est exempte d'huile insoluble dans le milieu hydrophile.

3. Composition selon l'une quelconque des revendications précédentes, caractérisée en ce que le pH est au plus égal à 3.

4. Composition selon l'une quelconque des revendications précédentes, caractérisée en ce que la quantité d'eau représente au plus 30% du poids total de la composition.

5. Composition selon l'une quelconque des revendications précédentes, caractérisée en ce que le composé à polyinsaturation oléfinique est le méthylène bis-acrylamide.

6. Composition selon l'une quelconque des revendications précédentes, caractérisée en ce que le copolymère est présent à raison de 0,01% à 5% du poids total de la composition.

7. Composition selon l'une quelconque des revendications précédentes, caractérisée en ce que le copolymère est présent à raison de 0,1% à 3% du poids total de la composition.

8. Composition selon l'une quelconque des revendications précédentes, caractérisée en ce que le milieu hydrophile contient au moins un solvant organique hydrophile choisi parmi les alcools mono- ou polyfonctionnels, les polyéthylène glycols éventuellement oxyéthylénés, les esters de propylène glycol, le sorbitol et ses dérivés, les di-alkyle d'isosorbide, les éthers de glycol et les éthers de propylène glycol.

9. Composition selon l'une quelconque des revendications précédentes, caractérisée en ce que le solvant hydrophile représente de 5 % à 90 % du poids total de la composition.

10. Composition selon l'une quelconque des revendications précédentes, caractérisée en ce qu'il contient au moins huile soluble dans le solvant.

11. Composition selon la revendication 10, caractérisée en ce que l'huile est une huile siliconée.

12. Composition selon la revendication 11, caractérisée en ce que l'huile est choisie parmi les cyclométhicones, les polydiméthylsiloxanes éventuellement oxyéthylénés et les phényldiméthylsiloxanes.

13. Composition selon l'une quelconque des revendications précédentes, caractérisée en ce que l'huile représente de 0 % à 50% du poids total de la composition.

14. Composition selon l'une quelconque des revendications précédentes, caractérisée en ce que l'huile représente de 1% à 15% du poids total de la composition.

15. Composition selon l'une quelconque des revendications précédentes, caractérisée en ce qu'elle contient, en outre, au moins un actif dépigmentant apte à dépigmenter la peau d'un être humain.

16. Composition selon l'une quelconque des revendications précédentes, caractérisée en ce qu'elle contient au moins un actif choisi parmi l'acide ascorbique, l'acide kojique, l'acide citrique, l'acide caféique, l'acide salicylique et ses dérivés, l'acide glucuronique, l'acide glycolique, l'acide pyruvique, l'acide 2-hydroxybutanoïque, l'acide 2-hydroxy-pentanoïque, l'acide 2-hydroxyhexanoïque, l'acide 2-hydroxyheptanoïque, l'acide 2-hydroxyoctanoïque, l'acide 2-hydroxynonanoïque, l'acide 2-hydroxydécanoïque, l'acide 2-hydroxyundécanoïque, l'acide 2-hydroxydodécanoïque, l'acide 2-hydroxytétradécanoïque, l'acide 2-hydroxyhexadécanoïque, l'acide 2-hydroxyoctadécanoïque, l'acide 2-hydroxytétraécosanoïque, l'acide 2-hydroxyeïcosanoïque, l'acide mandélique, l'acide benzoïque, l'acide phényllactique, l'acide gluconique, l'acide galacturonique, l'acide citrique, l'acide aleuritique, l'acide ribonique, l'acide tartronique, l'acide tartrique, l'acide malique, l'acide fumarique, l'acide rétinoïque et ses dérivés, l'acide benzène 1,4-di(3-méthylidène 10-camphosulfonique).

17. Composition selon l'une quelconque des revendications précédentes, caractérisée en ce qu'elle contient au moins un actif choisi parmi l'acide kojique, l'acide caféique, l'acide salicylique et ses dérivés, et leurs mélanges.

18. Composition selon l'une quelconque des revendications précédentes, caractérisée en ce qu'elle contient, encore, au moins un additif choisi parmi, les céramides, les agents conservateurs, les agents antioxydants, les agents émollients, les parfums.

19. Composition selon l'une quelconque des revendications précédentes, caractérisée en ce qu'elle consiste en une composition cosmétique et/ou dermatologique.

20. Utilisation non-thérapeutique de la composition selon l'une quelconque des revendications 1 à 19, pour traiter la peau du visage et/ou du corps humain.

21. Utilisation non-thérapeutique de la composition selon l'une quelconque des revendications 15 à 17, pour dépigmenter la peau du visage et/ou du corps humain.

22. Procédé de traitement cosmétique de la peau, caractérisé en ce qu'il consiste à appliquer sur la peau une composition selon l'une quelconque des revendications 1 à 19.

23. Procédé non-thérapeutique pour dépigmenter la peau du visage et/ou du corps humain, caractérisé en ce qu'il consiste à appliquer sur la peau une composition selon l'une quelconque des revendications 15 à 17.

24. Utilisation de la composition selon l'une quelconque des revendications 1 à 19, pour préparer une pommade ou un onguent destiné à traiter thérapeutiquement le visage et/ou le corps humain.

25. Utilisation d'un copolymère tel que défini dans l'une quelconque des revendications 1 à 19, pour solubiliser un actif dans une composition homogène macroscopiquement, contenant un milieu hydrophile acide, riche en solvant organique et présentant un pH au plus égal à 4, ce milieu renfermant une quantité d'eau représentant au plus 45% du poids total de la composition et/ou pour gélifier ladite composition.

## Claims

1. Composition containing an acidic hydrophilic medium and at least one gelling agent formed from a crosslinked anionic copolymer of acrylamide, of 2-acrylamido-2-methylpropanesulphonic acid and of at least one compound containing olefinic polyunsaturation which serves as a crosslinking agent, characterized in that the hydrophilic medium is a medium, rich in organic solvent, which has a pH of not more than 4, this medium containing an amount of water representing not more than 45% of the total weight of the composition, the gelling agent giving the composition a macroscopically homogeneous appearance.

2. Composition according to Claim 1, characterized in that the composition is free of insoluble oil in the hydrophilic medium.

3. Composition according to either of the preceding claims, characterized in that the pH is not more than 3.

4. Composition according to any one of the preceding claims, characterized in that the amount of water represents not more than 30% of the total weight of the composition.

5. Composition according to any one of the preceding claims, characterized in that the compound containing olefinic polyunsaturation is methylenebisacrylamide.

6. Composition according to any one of the preceding claims, characterized in that the copolymer is present in a proportion of from 0.01% to 5% of the total weight of the composition.

7. Composition according to any one of the preceding claims, characterized in that the copolymer is present in a proportion of from 0.1% to 3% of the total weight of the composition.

8. Composition according to any one of the preceding claims, characterized in that the hydrophilic medium contains at least one hydrophilic organic solvent chosen from mono- or polyfunctional alcohols, optionally oxyethylenated polyethylene glycols, propylene glycol esters, sorbitol and its derivatives, dialkyl isosorbides, glycol ethers and propylene glycol ethers.

9. Composition according to any one of the preceding claims, characterized in that the hydrophilic solvent represents from 5% to 90% of the total weight of the composition.

10. Composition according to any one of the preceding claims, characterized in that it contains at least one oil which is soluble in the solvent.

11. Composition according to Claim 10, characterized in that the oil is a silicone oil.

12. Composition according to Claim 11, characterized in that the oil is chosen from cyclomethicones, optionally oxyethylenated polydimethylsiloxanes and phenyldimethylsiloxanes.

13. Composition according to any one of the preceding claims, characterized in that the oil represents from 0% to 50% of the total weight of the composition.

14. Composition according to any one of the preceding claims, characterized in that the oil represents from 1% to 15% of the total weight of the composition.

15. Composition according to any one of the preceding claims, characterized in that it also contains at least one depigmenting active agent which is capable of depigmenting human skin.

16. Composition according to any one of the preceding claims, characterized in that it contains at least one active agent chosen from ascorbic acid, kojic acid, citric acid, caffeic acid, salicylic acid and its derivatives, glucuronic acid, glycolic acid, pyruvic acid, 2-hydroxybutanoic acid, 2-hydroxypentanoic acid, 2-hydroxyhexanoic acid, 2-hydroxyheptanoic acid, 2-hydroxyoctanoic acid, 2-hydroxynonanoic acid, 2-hydroxydecanoic acid, 2-hydroxyundecanoic acid, 2-hydroxydodecanoic acid, 2-hydroxytetradecanoic acid, 2-hydroxyhexadecanoic acid, 2-hydroxyoctadecanoic acid, 2-hydroxytetraecosanoic acid, 2-hydroxyeicosanoic acid, mandelic acid, benzoic acid, phenyllactic acid, gluconic acid, galacturonic acid, citric acid, aleuritic acid, ribonic acid, tartronic acid, tartaric acid, malic acid, fumaric acid, retinoic acid and its derivatives, and benzene-1,4-di(3-methylidene-10-camphorsulphonic acid).

17. Composition according to any one of the preceding claims, characterized in that it contains at least one active agent chosen from kojic acid, caffeic acid, salicylic acid and its derivatives, and mixtures thereof.

18. Composition according to any one of the preceding claims, characterized in that it also contains at least one additive chosen from ceramides, preserving agents, antioxidants, emollients and fragrances.

19. Composition according to any one of the preceding claims, characterized in that it consists of a cosmetic and/or dermatological composition.

20. Non-therapeutic use of the composition according to any one of Claims 1 to 19, for treating human facial and/or body skin.

21. Non-therapeutic use of the composition according to any one of Claims 15 to 17, for depigmenting human facial and/or body skin.

22. Cosmetic treatment process for the skin, characterized in that it consists in applying a composition according to any one of Claims 1 to 19 to the skin.

23. Non-therapeutic process for depigmenting human facial and/or body skin, characterized in that it consists in applying a composition according to any one of Claims 15 to 17 to the skin.

24. Use of the composition according to any one of Claims 1 to 19, to prepare a salve or an ointment intended for the therapeutic treatment of the human face and/or body.

25. Use of a copolymer as defined in any one of Claims 1 to 19, in order to dissolve an active agent in a macroscopically homogeneous composition, containing an acidic hydrophilic medium, which is rich in organic solvent and which has a pH of not more than 4, this medium containing an amount of water representing not more than 45% of the total weight of the composition, and/or in order to gel the said composition.

## Patentansprüche

1. Zusammensetzungen, die ein saures hydrophiles Medium und mindestens einen Gelbildner enthalten, der aus einem vernetzten, anionischen Copolymer von Acrylamid, 2-Acrylamino-2-methyl-propansulfonsäure und mindestens einer mehrfach olefinisch ungesättigten Verbindung, die als Vernetzungsmittel dient, gebildet wird, dadurch gekennzeichnet, daß es sich bei dem hydrophilen Medium um ein Medium handelt, das einen hohen Gehalt an organischem Lösungsmittel und einen pH-Wert von höchstens 4 aufweist, wobei dieses Medium eine Wassermenge enthält, die höchstens 45 % des Gesamtgewichts der Zusammensetzung ausmacht und wobei der Gelbildner der Zusammensetzung ein makroskopisch homogenes Aussehen verleiht.

2. Zusammensetzungen nach Anspruch 1, dadurch gekennzeichnet, daß die Zusammensetzungen kein in dem hydrophilen Medium unlösliches Öl enthalten.

3. Zusammensetzungen nach einem der vorhergehenden Ansprüche, dadurch gekennzeichnet, daß der pH-Wert höchstens 3 ist.

4. Zusammensetzungen nach einem der vorhergehenden Ansprüche, dadurch gekennzeichnet, daß die Wassermenge höchstens 30 % des Gesamtgewichts der Zusammensetzung ausmacht.

5. Zusammensetzungen nach einem der vorhergehenden Ansprüche, dadurch gekennzeichnet, daß die mehrfach olefinisch ungesättigte Verbindung Methylenbis(acrylamid) ist.

6. Zusammensetzungen nach einem der vorhergehenden Ansprüche, dadurch gekennzeichnet, daß das Copolymer in einem Mengenanteil von 0,01 bis 5 % des Gesamtgewichts der Zusammensetzung vorliegt.

7. Zusammensetzungen nach einem der vorhergehenden Ansprüche, dadurch gekennzeichnet, daß das Copolymer in einem Mengenanteil von 0,1 bis 3 % des Gesamtgewichts der Zusammensetzung vorliegt.

8. Zusammensetzungen nach einem der vorhergehenden Ansprüche, dadurch gekennzeichnet, daß das hydrophile Medium mindestens ein hydrophiles, organisches Lösungsmittel enthält, das unter monofunktionellen oder polyfunktionellen Alkoholen, gegebenenfalls ethoxylierten Polyethylenglykolen, Propylenglykolestern, Sorbit und seinen Derivaten, Dialkylisosorbiden, Glykolethern und Propylenglykolethern ausgewählt ist.

9. Zusammensetzungen nach einem der vorhergehenden Ansprüche, dadurch gekennzeichnet, daß das hydrophile Lösungsmittel 5 bis 90 % des Gesamtgewichts der Zusammensetzung ausmacht.

10. Zusammensetzungen nach einem der vorhergehenden Ansprüche, dadurch gekennzeichnet, daß sie mindestens ein Öl enthalten, das in dem Lösungsmittel löslich ist.

11. Zusammensetzungen nach Anspruch 10, dadurch gekennzeichnet, daß das Öl ein Siliconöl ist.

12. Zusammensetzungen nach Anspruch 11, dadurch gekennzeichnet, daß das Öl unter Cyclometiconen, gegebenenfalls ethoxylierten Polydimethylsiloxanen und Phenyldimethylsiloxanen ausgewählt ist.

13. Zusammensetzungen nach einem der vorhergehenden Ansprüche, dadurch gekennzeichnet, daß das Öl 0 bis 50 % des Gesamtgewichts der Zusammensetzung ausmacht.

14. Zusammensetzungen nach einem der vorhergehenden Ansprüche, dadurch gekennzeichnet, daß das Öl 1 bis 15 % des Gesamtgewichts der Zusammensetzung ausmacht.

15. Zusammensetzungen nach einem der vorhergehenden Ansprüche, dadurch gekennzeichnet, daß sie unter anderem mindestens einen depigmentierenden Wirkstoff enthalten, der befähigt ist, die menschliche Haut zu depigmentieren.

16. Zusammensetzungen nach einem der vorhergehenden Ansprüche, dadurch gekennzeichnet, daß sie mindestens einen Wirkstoff enthalten, der ausgewählt ist unter Ascorbinsäure, Kojisäure, Citronensäure, Kaffeesäure, Salicylsäure und ihren Derivaten, Glucuronsäure, Glykolsäure, Brenztraubensäure, 2-Hydroxybutansäure, 2-Hydroxypentansäure, 2-Hydroxyhexansäure, 2-Hydroxyheptansäure, 2-Hydroxyoctansäure, 2-Hydroxynonansäure, 2-Hydroxydecansäure, 2-Hydroxyundecansäure, 2-Hydroxydodecansäure, 2-Hydroxytetradecansäure, 2-Hydroxyhexadecansäure, 2-Hydroxyoctadecansäure, 2-Hydroxytetraeicosansäure, 2-Hydroxyeicosansäure, Mandelsäure, Benzoesäure, Phenylmilchsäure, Gluconsäure, Galacturonsäure, Citronensäure, Aleuritinsäure, Ribonsäure, Tartronsäure, Weinsäure, Äpfelsäure, Fumarsäure, Retinoesäure und ihren Derivaten sowie Benzol-1,4-di(3-methylidencampher-10-sulfonsäure).

17. Zusammensetzungen nach einem der vorhergehenden Ansprüche, dadurch gekennzeichnet, daß sie mindestens einen Wirkstoff enthalten, der unter Kojisäure, Kaffeesäure, Salicylsäure und ihren Derivaten und den Gemischen dieser Verbindungen ausgewählt ist.

18. Zusammensetzungen nach einem der vorhergehenden Ansprüche, dadurch gekennzeichnet, daß sie noch mindestens einen Zusatzstoff enthalten, der unter Ceramiden, Konservierungsmitteln, Antioxidantien, Emollentien und Parfums ausgewählt ist.

19. Zusammensetzungen nach einem der vorhergehenden Ansprüche, dadurch gekennzeichnet, daß sie aus kosmetischen und/oder dermatologischen Zusammensetzungen bestehen.

20. Nichttherapeutische Verwendung der Zusammensetzungen nach einem der Ansprüche 1 bis 19 zur Behandlung der Haut des Gesichts und/oder des menschlichen Körpers.

21. Nichttherapeutische Verwendung der Zusammensetzungen nach einem der Ansprüche 15 bis 17 zur Depigmentierung der Haut des Gesichts und/oder des menschlichen Körpers.

22. Verfahren zur kosmetischen Behandlung der Haut, dadurch gekennzeichnet, daß es darin besteht, auf die Haut eine Zusammensetzung nach einem der Ansprüche 1 bis 19 aufzutragen.

23. Nichttherapeutisches Verfahren zur Depigmentierung der Haut des Gesichts und/oder des menschlichen Körpers, dadurch gekennzeichnet, daß es darin besteht, auf die Haut eine Zusammensetzung nach einem der Ansprüche 15 bis 17 aufzutragen.

24. Verwendung der Zusammensetzungen nach einem der Ansprüche 1 bis 19 zur Herstellung einer Pomade oder einer Salbe, die zur therapeutischen Behandlung des Gesichts und/oder des menschlichen Körpers bestimmt ist.

25. Verwendung eines Copolymers nach einem der Ansprüche 1 bis 19 zum Solubilisieren eines Wirkstoffs in einer makroskopisch homogenen Zusammensetzung, die ein saures, hydrophiles Medium enthält, das einen hohen Gehalt an organischem Lösungsmittel und einen pH-Wert von höchstens 4 aufweist, wobei dieses Medium eine Wassermenge enthält, die höchstens 45 % des Gesamtgewichts der Zusammensetzung ausmacht, und/oder zum Gelieren dieser Zusammensetzung.
